# EUROPEAN PATENT SPECIFICATION

(11) **EP 0 222 279 B1**
(45) Date of publication and mention of the grant of the patent: **31.01.1996**
(21) Application number: 86115148.8
(22) Date of filing: 31.10.1986
(51) Int. Cl.: C12N 15/76, C12N 15/12, C12N 9/00, C12P 21/02

(54) **Production of active proteins containing cystine residues**
Produktion von Cystinreste enthaltenden aktiven Proteinen
Production de protéines actives contenant des résidus cystiniques

(30) Priority: 01.11.1985 CA 494456
(43) Date of publication of application: 20.05.1987
(73) Proprietor: Cangene Corporation, Mississauga Ontario L4V 1J7 (CA)
(72) Inventor: Garvin, Robert T., Toronto Ontario M6S 1J1 (CA); James, Eric, Etobicoke Ontario M8X 1G7 (CA)
(74) Representative: Perry, Robert Edward

(56) References cited:
- EP-A- 0 161 629
- EP-A- 0 179 449
- GENE, vol. 32, no. 1-2, December 1984, pages 21-30, Elsevier Science Publishers, Amsterdam, NL; G. GRAY et al.: "Synthesis of bovine growth hormone by Streptomyces lividans"
- GENE, vol. 29, no. 12, July/August 1984, pages 315-321, Elsevier Science Publishers, Amsterdam, NL; K.KENDALL et al.: "Cloning and expression of an extracellular-agarase gene from Streptomyces coelicolor A3(2) in Streptomyces lividans 66"

## Description

The present invention generally relates to a process of producing proteins having cystine residues as an integral and necessary portion of their active structure by cultivating genetically-engineered microorganisms.

The ability to manipulate genes into microorganisms has become commonplace. The organism upon which the bulk of prior art in genetic engineering is based is Escherichia coli (E. coli), a Gram-negative, non-sporulating bacillus of the family Enterobacteriaceae found in great numbers in the normal feces of man and animals. Using recombinant DNA techniques, the gene coding for the synthesis of a protein can be placed into E. coli, which will then synthesize that protein. The genetic material used may be either a native gene, in which case the protein produced will be natural; or a designed gene, in which case the protein produced may have unique or unusual properties not found in nature.

Repeated successful applications of specific techniques in the general field of recombinant DNA have established that it is possible to achieve expression of a heterologous genetic element in E. coli, expression in this instance being limited to translation of the genetic information into a linear covalently-bonded amino acid array (a polypeptide) having a primary sequence colinear with the structural portion of its source genetic element, observing the relationship that one codon of the structural genetic element is equal to one amino acid of its corresponding polypeptide. Such translation, subsequent to the introduction of genetic material, is taken to be the sum of natural cellular physiological events or processes which together result in the gene-directed production of a polypeptide of 'correct', e.g. coded-for, linear structure. Heterologous refers to the non-host origin (either natural or synthetic) of the genetic element.

The prior art has concentrated on methods of isolating DNA, of cutting it up and putting it back together in new or unique ways, and on methods of introducing these created DNA molecules into new organisms. Such recombinant DNA techniques (i.e. extracting DNA from organisms, digesting DNA with restriction enzymes, developing analytical methods to sequence DNA, isolating components of restriction endonuclease-cut DNA, ligating together isolated DNA restriction fragments or the restriction digests themselves with natural or created DNA vectors containing genetic elements competent to act as reference points for the decoding of the DNA, transforming host cells with the competent vectors containing ligated restriction fragments of DNA, and arranging growth conditions to favour those cells which contain the recombinant genetic elements) have assumed that the production of a functional protein using the information contained in DNA was only a problem in decoding the DNA. Once the correct primary amino acid structure had been created from the genetic material introduced into a host cell, it was assumed that the active form of the protein would automatically follow from the inexorable operation of natural and ubiquitous cellular physiological laws. This teaching is based upon the long-established Anfinsen Principle, a central tenet of molecular biology, which states that the information needed to specify the complex three-dimensional structure of a protein molecule is contained in its primary amino acid sequence. (Anfinsen, 1964; 1973).

The importance of functionality and active form can be appreciated from the realization that proteins play crucial roles in virtually all biological processes: enzymatic catalysis, transport and storage, coordinated motion, mechanical support, immune protection, the generation and transmission of nerve impulses, and control of growth, differentiation, and reproduction. The activity of protein molecules involved in all of these processes is a consequence of their conformation, i.e. their three-dimensional structure. The acquisition of the correct three-dimensional structure for a protein is therefore vital for it to assume its function.

Until now, for reasons stated above, the prior art has not addressed either functionality or active form as integral to the practice of genetic engineering, but has instead treated the acquisition of correct three-dimensional structure on an ad hoc basis. Practitioners of genetic engineering have assumed that correct gene expression would insure the correct three-dimensional structure in the coded-for protein, and have dealt with those instances when it did not as aberrations from this imagined general rule.

Many investigators have sought expression in E. coli and other prokaryotes of a eukaryotic gene coding for a secretory protein. As expressed in their chosen host organism, these normally soluble eukaryotic proteins were generally found as an insoluble intracellular product having none of their natural enzymatic, immunogenic, hormonal, or structural properties. Among the many rationalizations for these failures, none have recognized a metabolic reality of cytoplasmic protein expression in E. coli, namely, the inability of E. coli to facilitate the formation of disulphide bridges during cytoplasmic protein expression.

The importance of this observation to the practice of genetic engineering is clear if one realizes that the native structure of eukaryotic secretory proteins -- those proteins of most interest to genetic engineers, because of their high potential commercial value -- is almost universally disulphide bonded. The failure to achieve the expression from prokaryotes of eukaryotic proteins in their naturally active, disulphide-bonded form has severely limited commercial exploitation.

Accordingly, the present inventors sought to develop an expression system adequate for the production in active, disulphide-bonded form of eukaryotic secretory proteins. This required the identification of prokaryotic cells able to carry out the essential features of eukaryotic secretory protein expression, in particular, including the ability to facilitate the formation of disulphide bonds during the process of protein secretion. The inventors of the present invention also sought means, more effective than conventional means, for the expression of those genes which code for disulphide-bond containing proteins (DBCPs).

Gray et al, Gene 32, Nos. 1-2 (December 1984) pages 21-30, disclose the expression of active bovine growth hormone by Streptomyces lividans under the control of the regulatory region of the Streptomyces fradiae aph gene.

EP-A-0179449 discloses a process for secreting homologous non-disulphide-bonded protein from Streptomyces.

EP-A-0161629 describes the use of a signal sequence responsible for transporting a homologous disulphide-bonded product (amylase inhibitor) of translation to the cellular apparatus responsible for the secretion of disulphide-bonded proteins in Streptomyces species containing a signal peptidase.

According to the present invention, a DNA sequence encoding a eukaryotic protein having at least one cystine residue, comprises:
(a) a regulatory nucleotide sequence selected from a Streptomyces host, the regulatory sequence including a promoter sequence and a signal sequence responsible for sending a homologous product of translation to the cellular apparatus responsible for the secretion of disulphide-bonded proteins, operably linked to
(b) a second nucleotide sequence encoding a disulphide bond-containing heterologous eukaryotic protein;
   with the proviso that the regulatory sequence is not selected from a Streptomyces endoH gene.

The present invention also provides a replicable microbial expression vehicle capable of expressing a eukaryotic protein having at least one cystine residue, and including a DNA sequence as defined above. This vehicle may be introduced into a Streptomyces host. The host may be cultivated to produce an active eukaryotic protein, e.g. a protein having biological, hormonal, immunological or enzymatic activity.

The present invention allows the successful expression, in fully-functional form, of heterologous gene products having cystine residues as an integral and necessary portion of their active structure.

The cell or microorganism chosen as host should have the natural facility to produce extracellular proteins containing disulphide bridges.

In the preferred embodiment, the host cell or microorganism should preferably contain high levels of disulphide interchange enzyme (DIE) activity of a type characterised by, but not limited to, the activity of protein disulphide isomerase enzyme (EC 5.3.4.1).

The genetic element chosen for expression in the host of the heterologous gene coding for the DBCP should contain the nucleotide sequence corresponding to that portion of a native host gene responsible for sending the immediate product of translation to the cellular secretory apparatus, where the processing for secretion, including disulphide bond formation, takes place. This address portion is necessary for the expression in active form of heterologous genes coding for DBCPs. It can be conveniently identified from a determination of the structure of the genetic element coding for a secreted DBCP naturally present in the host organism. This address portion can be either a portion of the actual host gene or can be a synthetic sequence. In either case the regulatory sequence may be modified to optimise expression of the desired DBCP.

In the accompanying drawings:

Figure 1 represents various molecular structures which may be formed during the biological processing of precursor proteins leading to the formation of active human insulin. All of these forms are to be found subsequent to the translation by ribosomes of the immediate human insulin gene product, pre-proinsulin mRNA. The amino acids are represented by the single letter convention. The circled amino acids are enzymatic cleavage points which result in the removal of the C-peptide.

Figure 2 is a diagrammatic representation of a gene coding for the synthesis of a secreted protein which contains disulphide bonds as an essential architectural determinant of its biologically-active, three-dimensional conformation.

Figure 3 illustrates the identification and isolation of clones containing probe-positive genes.

A - Probe-positive signals from the original transformation plates.
B - Re-screening of signal-positive cells from A (above) for the isolation of putative probe-positive clones.

Figure 4 represents an agarose gel electrophoretic analysis of restriction digests of one particular set of plasmids isolated from putative protease gene-containing clones identified as in Figure 3.

Figure 5 represents the nucleic acid sequence and its derived amino acid sequence coding for part of CG4019 preproprotease (including the address and first 12 amino acids of the structural portion). Vertical letters are restriction endonuclease sites; the single letter convention has been used to represent amino acids. The arrow represents the leader peptidase processing site.

Figure 6 represents the nucleic acid sequence and its derived amino acid sequence coding for the first 12 amino acids of prochymosin preceded by the preproprotease leader sequence. Joining was through the NotI restriction site. Letter conventions are as detailed in Figure 5.

Figure 7 is a diagrammatic representation of the DNA constructs and procedures used to create a competency testing mixture. This mixture may contain the native gene of interest, coding for the production of an extracellular disulphide bond-containing protein. Its use identifies a competent cell using CG1085 as host.

Figure 8 illustrates the change in phenotype associated with transformation of strain CG1085 by a gene coding for the production of an extracellular disulphide bond-containing protease.

A - The phenotype of CG1085 on skim milk agar prior to transformation.
B - The phenotype of CG1085 on skim milk agar subsequent to transformation by pCG6, the extracellular protease gene-containing plasmid.

Figure 9 illustrates the phenotype of transformed CG1085 on CC agar. The halo demonstrates the presence of disulphide-bonded protease.

Figure 10 is a representation of plasmid pCG6 which contains the gene coding for the synthesis of the extracellular disulphide bond-containing protease from CG4019.

Figure 11 illustrates the characteristic restriction map of the 8.4 kbp BamHI insert of pCG6, approximately to scale, showing the location of the protease B structural gene.

Figure 12 is a representation of plasmid pCG63 which contains the gene construct coding for the biosynthesis of extracellular prochymosin.

### Description of the Preferred Embodiment

Previously, practitioners have not described a process whose exercise will insure the correct formation of disulphide bonds in a protein gene product produced by expression of the gene for a eukaryotic secretory protein in a prokaryotic host. Disulphide bonds are essential to the activity of many protein gene products whose functional three-dimensional structure requires their creation and maintenance. The importance of disulphide bonds can be appreciated from a consideration of the structure of the insulin molecule.

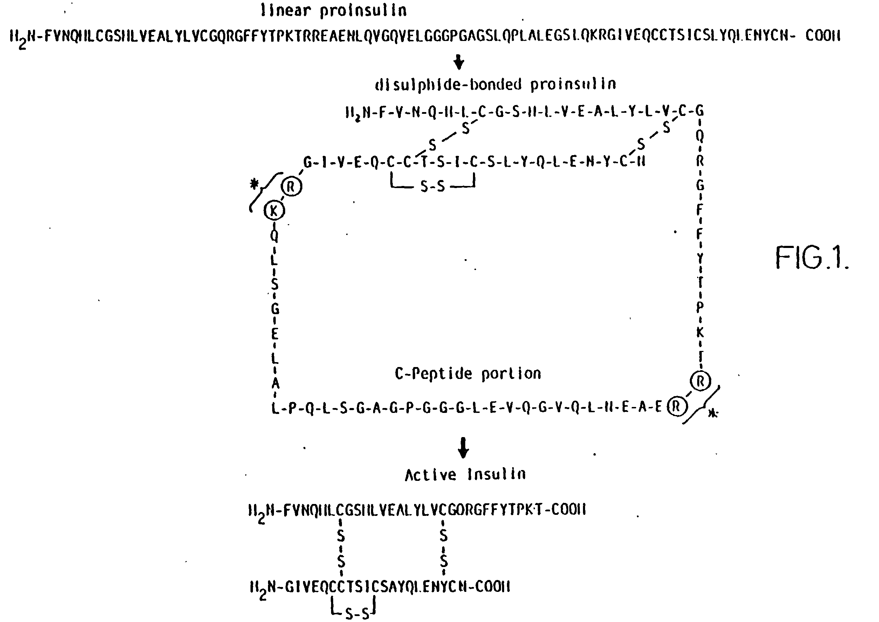

The amino acid sequence of human insulin is shown in Figure 1. Upon reduction of the disulphide bonds shown, the hormone loses its activity. In nature, insulin is formed from a precursor molecule, proinsulin (also shown in Figure 1), which is a single polypeptide chain having all of the necessary disulphide bonds, but containing an amino acid sequence not present in insulin. As indicated in Fig. 1, the removal of the connecting peptide (the C-peptide) by enzymatic digestion converts proinsulin, which is not active as a hormone, into active insulin. This enzymatic digestion occurs in the storage granules of the beta-cells of the pancreas. The beta-cell has the essential means to form the correct disulphide bridges required for folding the proinsulin molecule.

Using genetic engineering methodologies, the gene coding for human proinsulin has been introduced into E. coli. The commercial production of human insulin for therapeutic use has been hailed as the first success of genetic engineering in the health care sphere (Johnson, 1983). In contrast to expression in its natural host, however, proinsulin as expressed in E. coli collects in large aggregates called "inclusion bodies", a situation common to heterologous protein expression in E. coli (Williams, et al., 1982). Accordingly, before the E. coli-expressed proinsulin can be processed to the active insulin hormone, the disulphide bonds present in the natural molecule must be carefully put in place by exacting chemical techniques.

As a practical matter, procedures to "restore" disulphide bonds have had to be developed for a large number of genetically-engineered proteins whose genes have been placed into E. coli. This unexpected requirement has generally retarded the development of the field, and it is primarily the failure of E. coli to form disulphide bonds that has been responsible for the dearth of commercial products similar to human insulin to have issued from the practice of recombinant DNA genetic engineering.

it has been ascertained by the present inventors that the enzymatic activity of cytoplasmic proteins from genes of different prokaryotic genera are often preserved when the genes are cloned and expressed in E. coli, arguing that the "foreign" protein has found a familiar molecular milieu and therefore achieved an active structure.

The present inventors have also found that the thioredoxin system of E. coli, known to be active in the reduction of protein disulphides by NADPH [Holmgren, 1976; 1979; Holmgren, et al. 1978], had a similar counterpart throughout the prokaryotic and eukaryotic genera, arguing that reduction of disulphide bonds would be the norm in the cytoplasm of all cells. Therefore, genetic expression at this cytoplasmic address would be inappropriate for those proteins whose biological properties are disulphide bond determined.

A comprehensive survey of prokaryotes by the inventors revealed that high levels of DIE or DIE-like activity were rarely found. When it was found, however, it generally coincided with a striking ability of the host cell to secrete disulphide bonded protein(s).

No cytoplasmic proteins containing disulphide bonds have been found by the inventors either in E. coli or in certain examples of Bacillus, the expression host second only to E. coli in popularity with genetic engineers. Furthermore, while certain members of Bacillus secrete protein at high levels, the present inventors found that such strains did not secrete protein whose natural biological properties required a disulphide bonded structure. None of the E. coli or Bacillus strains assayed had significant levels of DIE or DIE-like activity. It is primarily for this reason that the present inventors have determined E. coli and Bacillus to be failures as expression hosts for eukaryotic secretory proteins: neither have the natural ability to facilitate the formation of disulphide bonds.

Of the eukaryotic proteins expressed in prokaryotes with the preservation of their desired native properties, all are either disulphide bond free, have had their disulphide bonds consciously restored in vitro by chemical means, or have undergone inadvertent disulphide bond restoration, essentially by oxidation upon exposure to air.

The present inventors have confirmed the importance of cellular address to the oxidation state of proteins. Since they found that oxidation state was also a critical determinant of the biological activity of proteins, it was concluded that the failure to develop expression systems specifically to facilitate the formation of disulphide bonds constituted a most serious and completely overlooked limitation to the practice of genetic engineering.

As a practical illustration of this invention, a biosynthetic bovine prochymosin gene (coding for a eukaryotic secretory protein containing disulphide bonds) has been cloned into a competent microorganism using an expression vector especially constructed so as to express heterologous protein extracellularly and in its natural, disulphide bonded form. The methods of identifying the host cell to be used and of constructing an appropriate expression vector are set forth. The implementation of this invention results in the expression of mammalian secretory proteins in a fermentable prokaryotic host at a level of production and at a cost to make them attractive commercial products. This invention therefore significantly augments and extends the use of recombinant DNA by practitioners of genetic engineering.

The development of the expression system of the present invention capable of expressing a heterologous DBCP extracellularly began by surveying existing organism collections and strains isolated from the environment for their extracellular protein production. This involved growing isolates in shake and fermentation cultures, removing the cells, and analyzing the supernatant culture fluid for secreted disulphide bonded proteins by high-performance liquid chromatography (HPLC) and sodium dodecylsulphate-polyacrylamide gel electrophoresis (SDS-PAGE).

Methods used for HPLC separation of extracellular proteins were those which resulted in little or no denaturation. For example, hydrophobic interaction HPLC (Kato, et al., 1984) was found to be useful for the separation of proteins in the range 5 - 50 kDal, independent of molecular weight and with a high recovery of activity, while size exclusion HPLC (Barden, 1983) was found useful for the separation of proteins in the range 20 - 100 kDal, according to molecular weight and with a high retention of native structure. SDS-PAGE was carried out according to Laemmli (1970).

Proteins purified in native form by HPLC from cellular supernatants were subjected to analysis of their disulphide bond content. Among the many methods available, the most generally useful procedure for this analysis was found to be that of Thannhauser, et al. (1984), in which the formation of 2-nitro-5-thiobenzoate from the reaction of 2-nitro-5-thiosulfobenzoate with disulphides in the presence of excess sodium sulfite is quantified.

In this way, those cellular isolates which naturally produce high levels of extracellular, disulphide bonded proteins can be determined.

The cellular isolates shown to produce high levels of disulphide bonded extracellular protein were analyzed for their DIE or DIE-like activity. Whole-cell homogenates can be assayed by a modification of the method of Ibbetson and Freedman (1976). Thus DBCP-excreting microbes found to contain high levels of DIE-like activity were considered candidates as ideal hosts for the economic production of heterologous secretory proteins having cystine as an essential structural feature.

The gene coding for the primary sequence of a polypeptide whose expression in its high DIE-containing host results in the production of high levels of disulphide-bonded extracellular protein may be isolated by the application of the following conventional protocol:
i) purification of the identified disulphide bonded protein by HPLC, electrophoresis or other standard procedures;
ii) partial amino acid sequencing of the protein to identify "runs" of 6 or 7 amino acids followed by analysis to determine which of the runs provide the least-redundant nucleic acid coding sequences;
iii) synthesis of the set of least redundant nucleic acid oligonucleotides corresponding to one or more of the determined amino acid runs to act as probes for gene isolation from an appropriate genomic library;
iv) identification of the gene for the targeted protein from a gene library constructed from the selected organism using the set of constructed oligonucleotides;
v) determination of the structure of the isolated gene by restriction analysis and nucleic acid sequencing.

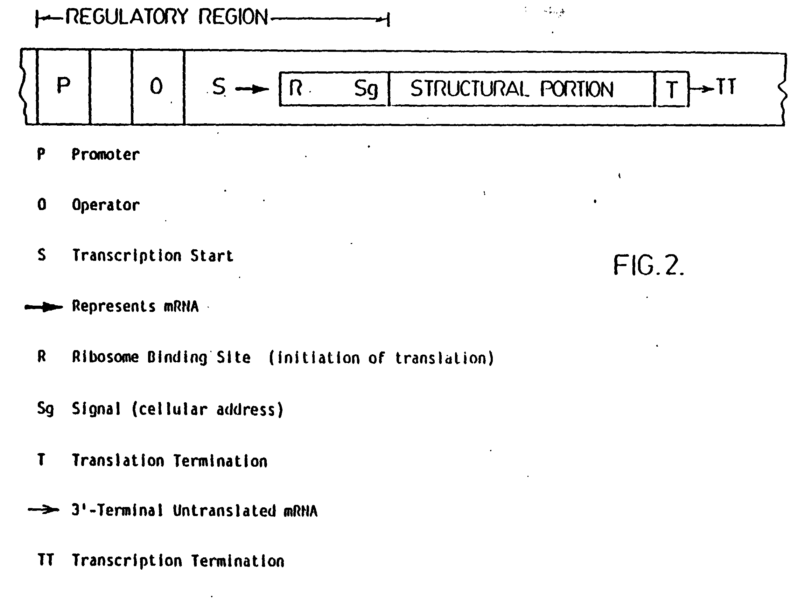

The most important part of the gene thus isolated and characterized, from an expression point of view, is the regulatory region (see Figure 2). This portion contains all of the genetic control signals used by the organism to synthesize, address, and process the resultant gene product. Most of these signals are host specific. The structural portion of the gene sequence is, however, not host-specific, and is subject only to the constraint of codon utilization. A heterologous structural gene can be spliced into the DNA sequence, once it is known, in such a way that the regulatory portion is effective in directing the synthesis, addressing, and processing of the desired gene product. The genetic element chosen for expression could be, for example, a eukaryotic secretory protein. In this case, the genetic expression element would comprise the structural portion of the eukaryotic gene joined to that portion of a gene coding for a disulphide bonded secretory protein native to the host which specifies extracellular address. The host, chosen because it contained all of the components required for the secretion of disulphide bonded proteins and utilizing the correct addressing portion of the gene, would thus ensure delivery of the desired gene product to the site appropriate for secretory processing.

The inserted structural gene sequence may be of natural origin or one synthesized in order to optimize expression and secretion by the chosen host. The synthetic gene may even code for an engineered protein not known in nature. This invention is therefore useful in general for the production of any secretory protein having disulphide bonds as a feature of the molecular structure necessary for its activity.

Best results are obtained when the regulatory portion of the gene sequence is optimized for expression of the structural portion by varying the junction region sequence around the initial codon of the structural gene. The precise sequence in this region is of importance for efficient excretion, processing and cystine formation.

As a specific example of the overall invention, a microorganism believed by the present inventors to be the Streptomyces griseus Type Strain [ATCC 23921] was identified as having DIE-like activity and of being able to produce an extracellular protease family, at least one member of which was found to contain disulphide bonds. One isolate, typical of a number of similar such isolates and designated CG4019, grew on CC agar medium with the production of a large, white halo surrounding the colony. The appearance of this halo was due to the secretion of a disulphide bonded protease. The gene coding for the synthesis and addressing of this disulphide bond-containing extracellular protease was isolated from CG4019 as follows.

Total DNA prepared from CG4019 as described by Chater et al. (1982) was digested with BamHI and then ligated into the BamHI site of pBR322 (Bolivar et al., 1977), thus creating a CG4019/BamHI gene library. [Unless otherwise noted, molecular cloning procedures utilize the protocols of Maniatis et al. (1982)]. DNA from the CG4019/BamHI gene library was used to transform E. coli HB101 (Boyer and Roulland-Dussoir, 1969) selecting for resistance to ampicillin. The transformants as a group contain, as BamHI inserts in pBR322, the total genome of CG4019.

To identify which transformant clones contain the gene for the disulphide bond-containing extracellular protease, DNA isolated from each transformant was hybridized to a special oligonucleotide probe. The nucleic acid sequence of the special hybridization probe was based on "runs" of amino acids present in the CG4019 extracellular disulphide-bond containing protease protein (see Footnote 1, below).

One skilled in the art can follow the procedure as detailed here to probe the DNA structure of each transformant. Filter paper (Whatman 540) can be placed on top of the transformant clones after they have grown out on ampicillin plates following their transformation with DNA from the CG4019/BamHI gene library. Ink line-up marks made at this time allow the orientation of the paper with respect to its plate of origin to be made at some later date. After five minutes, the paper can be lifted off, placed colony side up onto filter paper presoaked with 0.2 N sodium hydroxide, 1.5 M sodium chloride, and allowed to sit for ten minutes. The paper can then be transferred to filter paper presoaked with 1.0 M. Tris, 3 M sodium chloride, pH 7, allowed to sit for ten additional minutes, and then washed by submersion with 2XSSC solution [Note: 20XSSC is 3 M sodium chloride, 0.3 M sodium citrate, pH 7], after which it should also be washed by submersion with 95% ethanol, air dried, and baked for two hours at 85 degrees Celsius.

These baked papers contain at least a sample of the DNA present in the original transformant clones localized in a position which can be related through the ink orientation marks back to the plate of origin, which itself should be incubated overnight after the transformant colonies have been transfered to the paper.

In order to identify that sample of DNA on the paper which contains gene sequences corresponding to the disulphide bond-containing extracellular protease gene sequence from CG4019, conditions should be arranged such that if a DNA sample hybridizes to a synthetic oligonucleotide having a unique protease sequence, then that sample is made visible.

To do this, the baked filters should first be washed with 6XSSC containing Denhardt's solution (see Maniatis et al., 1982, p. 327) and 0.5% sodium dodecyl sulphate (SDS) for forty-five minutes at 65 degrees Celsius, then polyriboadenylic acid (5 ug/ml), DNA (sonicated E. coli DNA, 50 ug/ml), and radioactive probe¹ (at least 160,000 cpm/ml) added. After hybridization for at least ten hours, the paper should be washed twice for at least thirty minutes each time with 3XSSC containing 0.5% SDS, and allowed to air dry.
1. Sixteen 18-mer deoxyoligonucleotide probes were synthesized and end-labelled with (32)-phosphorous using the kinasing procedure of Maniatis et al.(1978). The resulting sixteen sequences constitute all of the possible oligonucleotide sequences coding for the amino acid "runs" leu-thr-ala-ala-his-cys and leu-thr-ala-gly-his-cys present in the disulphide-bond-containing extracellular protease found to be present in CG4019.

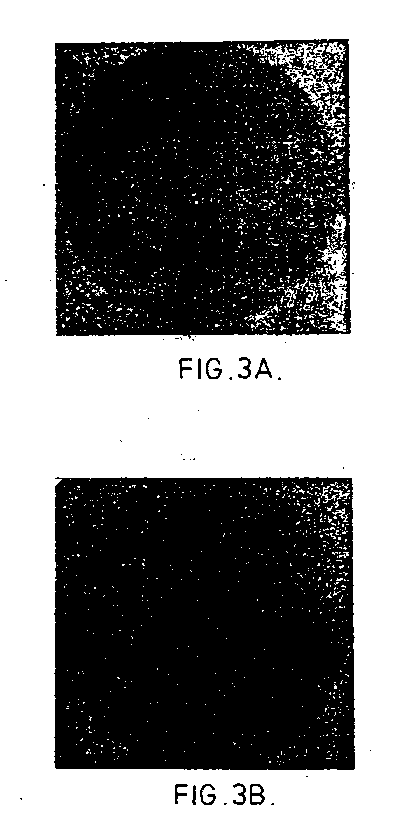

Those DNA samples on the paper representing clones that contain the protease gene will hybridize to one of the probes and would therefore be radioactive, while those DNA samples which do not contain protease gene sequences would probably not bind any of the unique protease probes. In order to determine which of the DNA samples following hybridization to the probe family are radioactive, the dried papers can be exposed to X-ray film following the procedures detailed in Maniatis et al.(1982). By correctly orienting each developed X-ray film containing positive signals with its plate of origin containing live cells, the set of clones which singly or together are likely to contain the disulphide-bond-containing extracellular protease gene of CG4019 (or its equivalent strain) can be identified (see Figure 3A).

A plug of agar containing cells from the region of the plate giving rise to probe positive hybridization signals can be used to inoculate medium, and the procedure repeated using plates containing now well-isolated single colonies. In this way, that set of clones likely to contain the probe-specific gene can be identified (see Figure 3B).

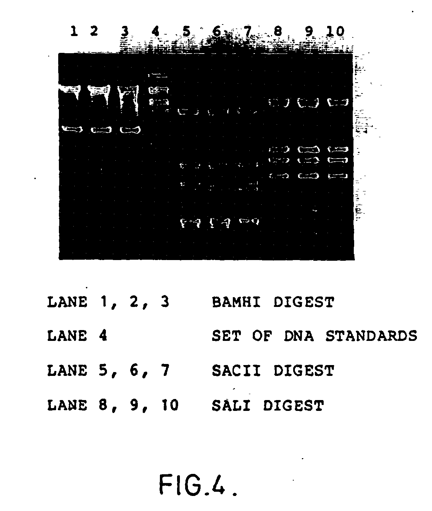

The BamHI insert of the contained plasmids in this set of putative protease gene containing clones thus isolated can be restriction analysed in order to determine the number of different inserts present in the set. Figure 4 shows a typical gel pattern from which it may be deduced that plasmids isolated from this set of putative clones all contained the same 8.4 kbp Bam/Bam insert. That the Bam/Bam insert contains the probe-specific DNA was deduced from a Southern blot of the gel pattern reported in Figure 4. One skilled in the art can sequence the DNA of each different BamHI insert in order to obtain a linear base array whose general characteristic structural elements, as indicated in Figure 2, may be deduced.

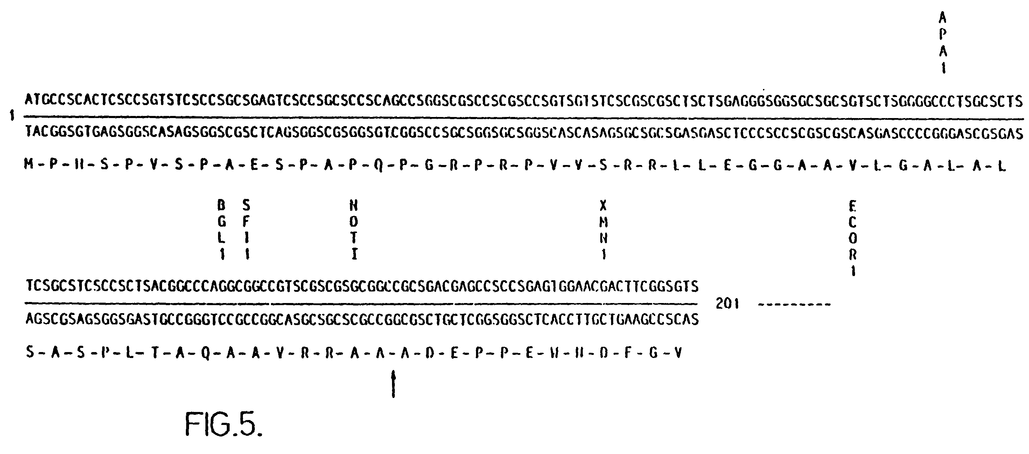

In this way, the signal portion (Sg in Figure 2) of the gene coding for the extracellular disulphide-bond-containing protease of CG4019 may be determined. The nucleotide sequence of the signal portion of such a gene is shown in Figure 5, which discloses the nucleic acid sequence from the start of translation of the addressing portion of a protease gene -- corresponding in protein structural terms to the pre portion of the molecule -- through the first thirty-six bases of its structural gene portion, corresponding to the first twelve amino acids of its pro protein portion. As can be seen in Figure 5, this portion of the gene sequence includes a NotI restriction site.

This sequence information concerning the addressing and structural portions of the gene coding for an extracellular disulphide bond-containing protease of CG4019 may be utilized to construct a calf prochymosin gene suitable for expression and secretion of calf prochymosin in active form from Streptomyces as follows.

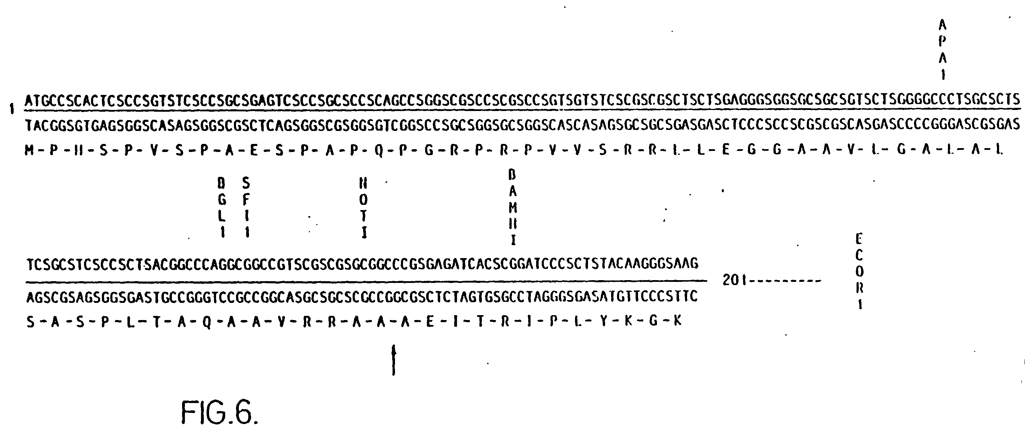

A synthetic prochymosin gene may be constructed having codons optimized for expression in Streptomyces and containing a 5'-end capable of ligating into a NotI restriction site and a 3'-end capable of ligating into some other convenient restriction site. Such a construct would preserve the natural Streptomyces protease signal sequence when it was ligated into the NotI site as shown in Figure 6. It may be determined that upon translation of such a genetic construct in the correct context, a preprochymosin molecule will be made whose cellular address is identical to that of the authentic preproprotease molecule.

The correct context for translation of such a preprochymosin genetic construct may be assured by employing a competent host cell which: (1) is able to produce excreted disulphide-bonded protein(s) utilizing the addressing portion of the preproprotease genetic element; and (2) harbours a host cell-compatible plasmid containing the preprochymosin genetic element constructed as detailed above. As a specific example, such a competent host cell was selected by the present inventors using the following protocol.

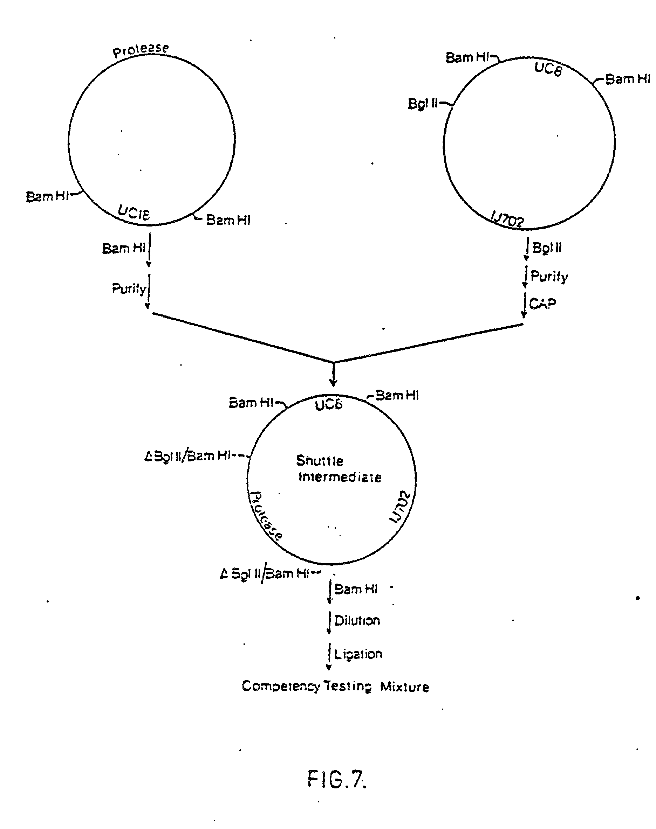

The BamHI inserts of the set of putative CG4109 protease gene-containing clones identified earlier by colony-blot hybridization were introduced into a Streptomyces-compatible vector by application of the recombinant DNA techniques outlined in Figure 7. The shuttle intermediates were grown up in the HB101 strain of E. coli and treated as shown in Figure 7 to prepare a competency testing mix containing two plasmids: a pIJ702/protease gene-containing one having only Streptomyces DNA, and pUC8 containing E. coli DNA. Both plasmids, because they came from HB101, were unmodified.

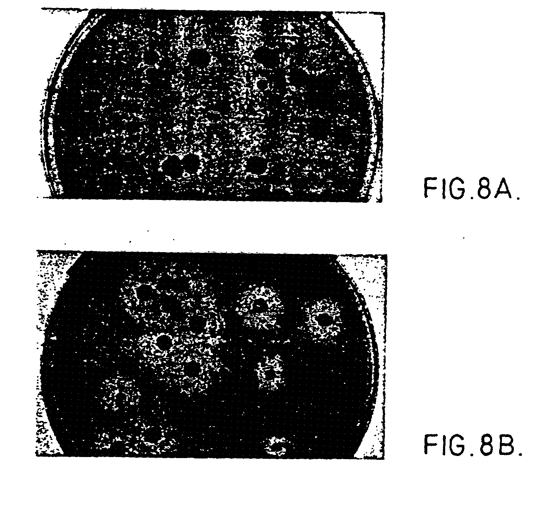

The competency-testing mix was used to transform various Streptomyces strains chosen, isolated, or otherwise known to: (1) have DIE-like activity; (2) produce extracellular disulphide bond-containing proteins (DBCP); and (3) be negative for extracellular protease. One such strain, a microorganism believed by the present inventors to be the Streptomyces lividans Type Strain [ATCC 23921], was identified by the present inventors to be thus potentially competent. One isolate of this strain, typical of a number of similar such isolates and designated CG1085, had the protease-negative phenotype on milk plates as shown in Figure 8A.

Transformation of this strain by the competency-testing mix (prepared as in Figure 7) gave rise to colonies exhibiting, on skim milk agar medium, the phenotype depicted in Figure 8B.

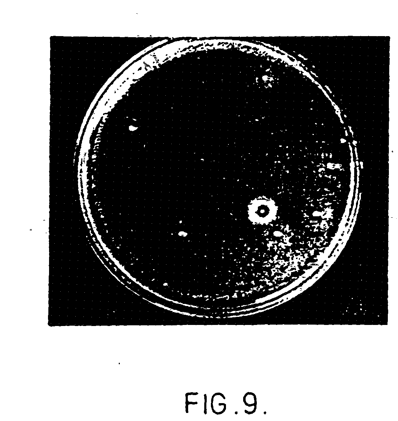

The hydrolysis surrounding the colony is produced by the action of extracellular protease on casein present in the medium. In a similar way, colonies of S. lividans transformed by the extracellular protease gene produce "halos" on CC agar as shown in Fig. 9. This biological activity, in the case of the protease genes isolated from CG4019, is known by the inventors herein to be dependent on the presence of disulphide bonds. The appearance of this phenotype after transformation by competency-testing mix identifies a competent host: able to secrete, in active form, a gene product whose location is extracellular and whose biological function is disulphide-bond dependent. In addition, the use of competency-testing mix identifies those potential host cells able to accept incoming DNA prepared as outlined in Figure 7, i.e., its use eliminates DNA restriction problems commonly encountered when wild-type isolates are used as DNA hosts.

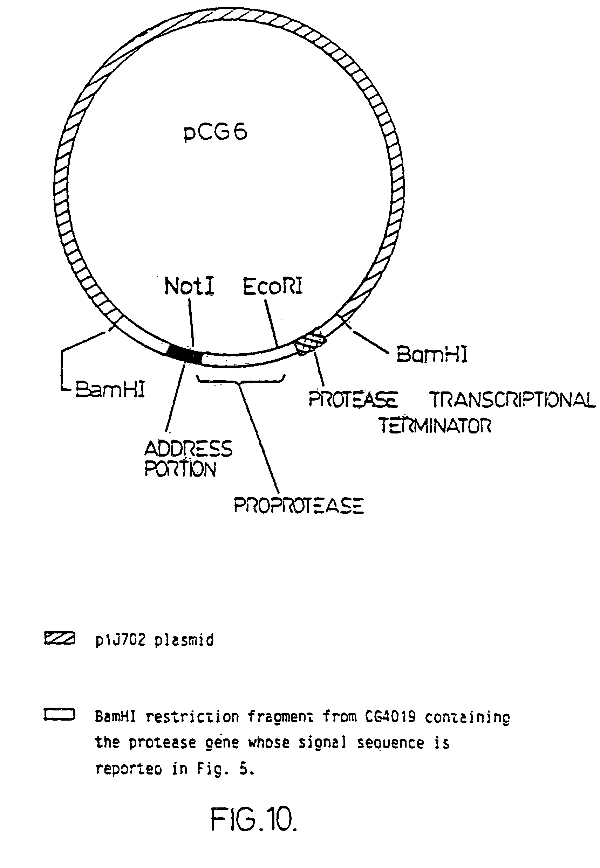

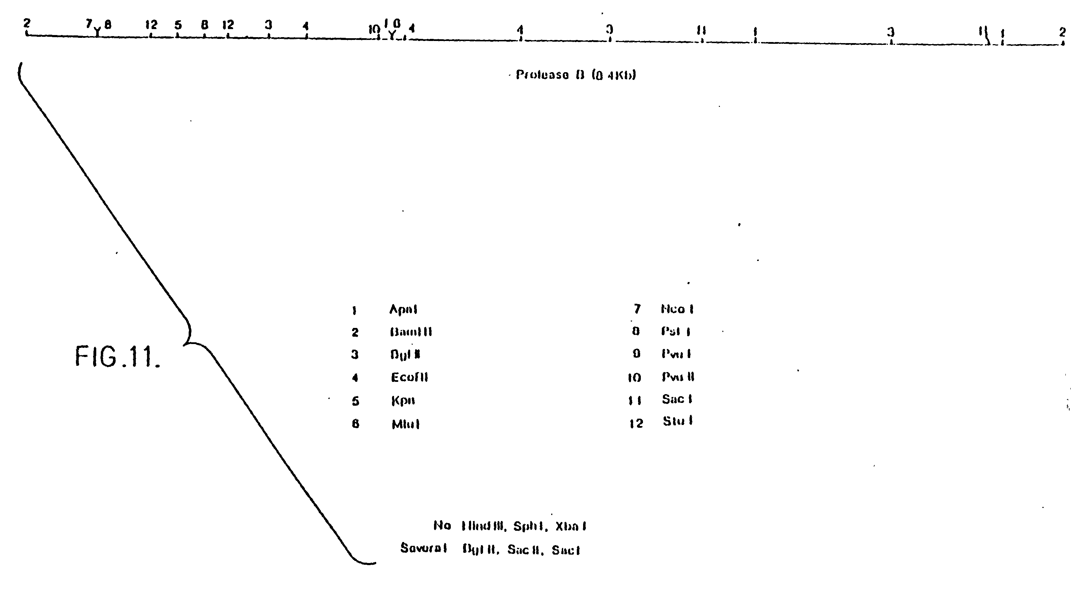

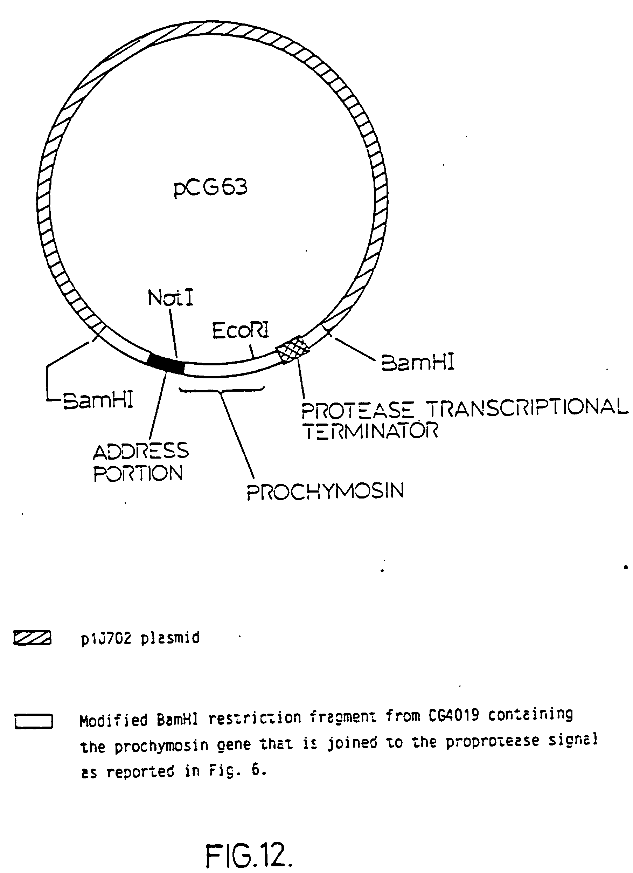

Plasmid isolated from clones thus identified as competent contain a functional genetic element coding for an extracellular protein having disulphide bonds as an essential architectural determinant. A portion of this genetic element must therefore specify an extracellular address. Figure 10 depicts the general structure of one such plasmid, pCG6, isolated from CG1085 transformed with protease gene-containing competency-testing mix, and characterized as having the structure shown. The characteristic restriction map of the BamHI insert of cPG6 is shown in Figure 11. The address portion of pCG6, whose sequence is shown in Figure 5, may be used to construct a plasmid coding for the extracellular production of biologically-active prochymosin protein. One such suitable construct able to transform CG1085 into an extracellular prochymosin-producing strain is shown in general in Figure 12, with the particular address portion and joining sequence as shown in Figure 6.

Upon transformation of CG1085 by a preprochymosin gene-containing plasmid of structure such as shown in Figure 12, a preprochymosin molecule will be made with cellular address identical to that of preproprotease. The host protein-processing system present at this address treats the preprochymosin molecule as it would preproprotease, thus processing it correctly by removing the pre portion of the molecule and forming the requisite disulphide bonds as the resultant functional prochymosin molecule is secreted outside the cell.

The central tenet of the genetic engineering process of the present invention is that the disulphide bond-containing heterologous secretory protein whose expression in fully-active form was sought (prochymosin in the preferred embodiment) would be achieved only by utilization of a host cell and genetic construct, chosen in the first instance and created in the latter, according to the teachings of this disclosure, and that such protein, whose secretion was achieved by following these teachings, would be in its natural disulphide-bonded conformation.

One may determine, by SDS-PAGE analysis (Laemmli, 1970) of concentrated cell-free culture supernatants of CG1085/pCG63, that the prochymosin-sized protein bands are Western blot-positive for prochymosin. Further, the application of the HPLC and disulphide assay procedures described earlier may show that a protein, isolated by HPLC from the cell-free culture supernatant of CG1085/pCG63 and shown to have a mobility identical to that of authentic calf prochymosin, contains disulphide bonds. It may be further determined that a genetic construct composed of the BamHI insert of pCG63 cloned into the BamHI site of pBR322 and used to transform E. coli HB101 results in no such production of extracellular material using identical analytical procedures.

For greater certainty, it will be apparent to one skilled in the art that the structural portion of any natural gene sequence -- or perhaps preferably any synthetic structural gene sequence (so codon utilization may be optimized) -- may be utilized in the expression vector and host which together constitute this invention. Such use, when coupled by the teachings of this invention to the regulatory region of a gene coding for a cystine-containing secretory protein from any microorganism source identified and developed as taught by the disclosures herein, will result in the production of any desired natural or designed disulphide-bonded protein, such as, but not limited to, eukaryotic secretory enzymes such as prochymosin, trypsins, amylases, ligninases, chymosin, lipases, cellulases; prokarytic secretory enzymes such as glucose isomerase, amylases, lipases, pectinases, cellulases, proteinases, oxidases, ligninases; blood factors such as Factor VIII and Factor VIII-related biosynthetic blood coagulant proteins, Factor IX; tissue-type plasminogen activator; hormones such as proinsulin; lymphokines such as beta and gamma-interferon, interleukin-2; enzyme inhibitors such as extracellular proteins containing disulphide bonds whose action is to destroy antibiotics either enzymatically or by binding, for example, B-lactamase inhibitor, a-trypsin inhibitor; growth factors such as nerve growth factors, tissue necrosis factors, colony stimulating factors; immunoglobulin-related molecules such as synthetic, designed, or engineered antibody molecules; cell receptors such as cholesterol receptor; viral antigens such as viral hemagglutinins, AIDS antigen and immunogen, hepatitis B antigen and immunogen, foot-and-mouth disease virus antigen and immunogen; bacterial surface effectors such as Protein A; toxins such as protein insecticides, algicides, fungicides, and biocides; systemic proteins of medical importance such as myocardial infarct protein (MIP), weight control factor (WCF), calloric rate protein (CRP).

The examples given above are limited to those natural secretory proteins having biological properties whose exercise is a consequence of the maintenance of their disulphide-bonded structure.

One skilled in the art can easily determine whether the use of any known or unknown organism will be within the scope of this invention by following the teaching of this disclosure.

The following species from the genus Streptomyces are identified as particularly suitable: acidophillus, albus, amylolyticus, argentiolus, aureofaciens, aureus, candidus, cellostaticus, cellulolyticus, coelicolor, creamorus, diastaticus, farinosus, flaveolus, flavogriseus, fradiae, fulvoviridis, fungicidicus, gelaticus, glaucescens, globisporus, griseolus, griseus, hygroscopicus, ligninolyticus, lipolyticus, lividans, moderatus, olivochromogenus, parvus, phaeochromogenes, plicatus, proteolyticus, rectus, roseolus, roseoviolaceus, scabies, thermolyticus, tumorstaticus, venezuelae, violaceus, violaceus-ruber, violascens, and viridochromogenes.

The genetically-engineered organism described in the preferred embodiment of this invention may be cultivated by growth in batch or continuous fermentation mode in a fermenter or by immobilization with carrier beads in a bioreactor. As a consequence of the genetic engineering principles described in this application applied to organisms of unique metabolism identified as taught herein, the desired heterologous secretory protein may be excreted out from the microorganism into the media in its natural conformation. The highest levels of active excreted protein are associated with those microorganisms having the highest levels of disulphide interchange enzyme or disulphide interchange enzyme-like activity.

The exercise of this invention thus avoids the difficult and often expensive task of chemical renaturation which has heretofore been necessary for the production of naturally-active cystine containing protein.

The publications and other materials referred to herein to illuminate the background of the invention and, in particular cases, provide additional detail respecting its practise are incorporated herein by reference and for convenience are numerically referred and grouped in the appended bibliography.

### RECIPES

### Skim Milk Agar

### Ingredients per litre:

| | |
|---|---|
| Skim milk powder (No Name) 8% | 80 gms |
| Bacto Nutrient Agar (Difco pg 619): | |
| Bacto beef extract | 3.0 gms |
| Bacto peptone | 5.0 gms |
| Bacto agar | 15.0 gms |

Final pH 6.8 ± 0.2 at 25°C

Dissolve skim milk powder and nutrient agar separately. Autoclave 500 ml media in 2 litre flasks 15 min at 121°C. Avoid overheating as caramelization will occur.

Then pour milk suspension into agar media. Flame plates to remove bubbles.

Plates are incubated and examined periodically for a clear zone of hydrolysis surrounding the bacterial growth.

Skim milk powder (No Name). The composition of this skim milk powder is: Skim milk powder, Vitamin A Palmitak and Vitamin D, 2200 I.U. Vitamin A per 100 g, 440 I.U. Vitamin D per 100 g.

Approximate nutritional analysis of one quart liquid (1.13 ltr) of No Name Sunfresh Ltd. skim milk powder is:

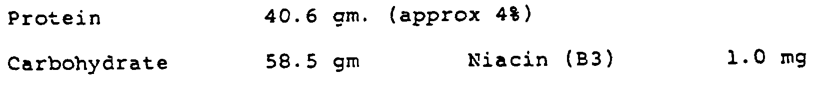

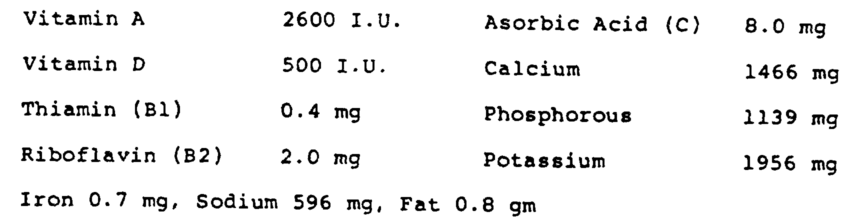

### CC Agar

### Ingredients per litre:

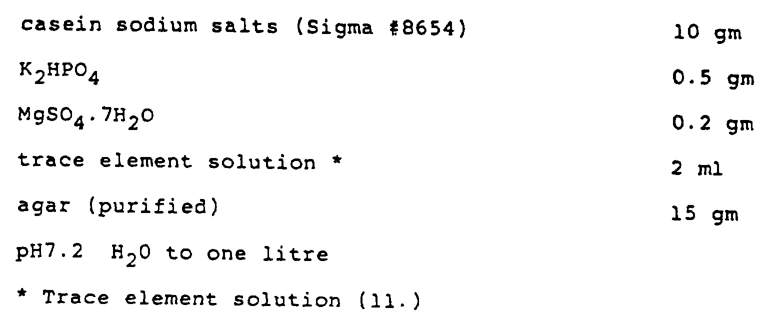

### Autoclave separately

| | |
|---|---|
| ZnCl₂ | 40 mg |
| FeCl₃.6H₂O | 200 mg |
| CuCl₂.2H₂O | 10 mg |
| MnCl₂.4H₂O | 10 mg |
| Na₂B₄O₇.10H₂O | 10 mg |
| (NH₄)₆Mo₇O₂₄.4H₂O | 10 mg |

H₂O to 1 ltr.

### REFERENCES

1. Anfinsen, C.B., "On the possibility of predicting tertiary structure from primary sequence", in New Perspectives in Biology [Sela, M. (ed.), American Elsevier, 1964] p.p. 42-50.
2. Anfinsen, C.B., "Principles that govern the folding of protein chains", Science 181:223-230 (1973).
3. Johnson, I.S., "Human Insulin from Recombinant DNA Technology", Science 219:632-637 (1983).
4. Williams, D.C., Van Frank, R.M., Muth, W.L., and Burnett, J.P., "Cytoplasmic inclusion bodies in Escherichia coli producing biosynthetic human insulin proteins", Science 215:687-689 (1982).
5. Holmgren, A., Proc. Natl. Acad. Sci. U.S.A., "Hydrogen donor system for Escherichia coli ribonucleosidediphosphate reductase dependent upon glutathione", 72:2275-2279 (1976).
6. Holmgren, A., "Gluthathione-dependent synthesis of deoxyribonucleotides", J. Biol. Chem. 254:3672-3678 (1979).
7. Holmgren, A., Ohlsson, I., & Grankvist, M.L., "Thioredoxin from Escherichia coli", J. Biol. Chem. 253:430-436 (1978).
8. Kato, Y., Kitamura, T., & Hashimoto, T., "New support for hydrophobic interaction chromatography of proteins", J. Chromatogr. 292:418-426 (1984).
9. Barden, J.A. "Separation of both fibrous and globular proteins on the basis of molecular weight using high-performance size exclusion chromatography" Anal Biochem. 135:52-57 (1983).
10. Thannhauser, T.W., Konishi, Y., Sheraga, H.A., "Sensitive quantitative analysis of disulphide bonds in polypeptides and proteins", Anal. Biochem. 138:181-188 (1984).
11. Ibbetson, A.L., & Freedman, R.B., "Thiol-Protein Disulphide Oxidoreductases" Biochem., J. 159:377-384 (1976).
12. Laemmli, U.K., "Cleavage of structural proteins during the assembly of the head of the bacteriophage T4", Nature 227:680-685 (1970).
13. Chater, K.F., Hopwood, D.A., Kieser, T., & Thompson, C.J. "Gene cloning in Streptomyces", Curr. Top. Microbiol. Immunol. 96:69-95 (1982).
14. Bolivar, F., Rodriguez, R.L., Greene, P.J., Betlach, M.C., Heyneker, H.L., Boyer, H.W., "Construction and characterization of new cloning vehicles II. A multipurpose cloning system", Gene 2:95-113 (1977).
15. Boyer, H.W., and Roulland-Dussoix, D., "A complementation analysis of the restriction and modification of DNA in Escherichia coli", J.M. Biol. 41:459-472 (1969).
16. Maniatis, T., Fritsch, E.F., and Sambrook, J. (Eds.), Molecular Cloning: a Laboratory Manual, Cold Srping Harbour Laboratory, 1982.
17. Maniatis, T., Hardison, R.C., Lacy, E., Lauer, J., O'Connell, C., Quon, D., Sim, G.K., and Efstratiadis A., "The isolation of structural genes from libraries of eucaryotic DNA", Cell 15:687-701 (1978).

## Claims (Claims for the following Contracting State(s): BE, CH, DE, FR, GB, GR, IT, LU, NL, SE)

1. A DNA sequence encoding a eukaryotic protein having at least one cystine residue, comprising:
(a) a regulatory nucleotide sequence selected from a Streptomyces host, the regulatory sequence including a promoter sequence and a signal sequence responsible for sending a homologous product of translation to the cellular apparatus responsible for the secretion of disulphide-bonded proteins, operably linked to
(b) a second nucleotide sequence encoding a disulphide bond-containing heterologous eukaryotic protein;
with the proviso that the regulatory sequence is not selected from a Streptomyces endoH gene.

2. The DNA sequence of claim 1, wherein said Streptomyces host is selected from the species acidophillus, albus, amylolyticus, argentiolus, aureofaciens, aureus, candidus, cellostaticus, cellulolyticus, coelicolor, creamorus, diastaticus, farinosus, flaveolus, flavogriseus, fradiae, fulvoviridis, fungicidicus, gelaticus, glaucescens, globisporus, griseolus, griseus, hygroscopicus, ligninolyticus, lipolyticus, lividans, moderatus, olivochromogenus, parvus, phaeochromogenes, plicatus, proteolyticus, rectus, roseolus, roseoviolaceus, scabies, thermolyticus, tumorstaticus, venezuelae, violaceus, violaceus-ruber, violascens or viridochromogenes.

3. The DNA sequence of claim 1, wherein the disulphide bond-containing heterologous eukaryotic protein is heterologous with respect to said Streptomyces host and is a natural or designed protein selected from the group consisting of prochymosin, trypsins, amylases, ligninases, chymosin, lipases, cellulases; glucose isomerase, pectinases, proteinases, oxidases; Factor VIII and Factor VIII-related biosynthetic blood coagulant proteins, Factor IX; Tissue-type plasminogen activator; proinsulin; gamma-interferon, interleukin-2; extracellular proteins containing disulphide bonds whose action is to destroy antibiotics either enzymatically or by binding, β-lactamase inhibitor, α-trypsin inhibitor; nerve growth factors, tissue necrosis factors, colony-stimulating factors; synthetic, designed or engineered antibody molecules; cholesterol receptor; viral hemagglutinins, AIDS antigen and immunogen, hepatitis B antigen and immunogen, foot-and-mouth disease virus antigen and immunogen; protein A; protein insecticides, algicides, fungicides, and fungicides; Myocardial Infarct Protein (MIP), Weight Control Factor (WCF) or Calloric Rate Protein (CRP).

4. The DNA sequence of claim 1, wherein the regulatory sequence also includes an operator sequence, a transcription-start sequence, and a ribosome-binding site sequence.

5. A replicable microbial expression vehicle capable of expressing an eukaryotic protein, wherein said eukaryotic protein has at least one cystine residue and has a DNA sequence according to any preceding claim.

6. A Streptomyces host harbouring a replicable microbial expression vehicle according to claim 5.

7. The Streptomyces host of claim 6, which has a disulphide-interchange enzyme or disulphide-interchange enzyme-like activity.

8. A process for producing an active eukaryotic protein that has at least one cystine residue, said process comprising cultivating the Streptomyces host of claim 6.

9. The process of claim 8, wherein said active eukaryotic protein has biological, hormonal, immunological or enzymatic activity.

## Claims (Claims for the following Contracting State(s): AT, ES)

1. A method for preparing a DNA sequence encoding a eukaryotic protein having at least one cystine residue, comprising operably linking:
(a) a regulatory nucleotide sequence selected from a Streptomyces host, the regulatory sequence including a promoter sequence and a signal sequence responsible for sending a homologous product of translation to the cellular apparatus responsible for the secretion of disulphide-bonded proteins, to
(b) a second nucleotide sequence encoding a disulphide bond-containing heterologous eukaryotic protein;
with the proviso that the regulatory sequence is not selected from a Streptomyces endoH gene.

2. The method of claim 1, wherein said Streptomyces host is selected from the species acidophillus, albus, amylolyticus, argentiolus, aureofaciens, aureus, candidus, cellostaticus, cellulolyticus, coelicolor, creamorus, diastaticus, farinosus, flaveolus, flavogriseus, fradiae, fulvoviridis, fungicidicus, gelaticus, glaucescens, globisporus, griseolus, griseus, hygroscopicus, ligninolyticus, lipolyticus, lividans, moderatus, olivochromogenus, parvus, phaeochromogenes, plicatus, proteolyticus, rectus, roseolus, roseoviolaceus, scabies, thermolyticus, tumorstaticus, venezuelae, violaceus, violaceus-ruber, violascens or viridochromogenes.

3. The method of claim 1, wherein the disulphide bond-containing heterologous eukaryotic protein is heterologous with respect to said Streptomyces host and is a natural or designed protein selected from the group consisting of prochymosin, trypsins, amylases, ligninases, chymosin, lipases, cellulases; glucose isomerase, pectinases, proteinases, oxidases; Factor VIII and Factor VIII-related biosynthetic blood coagulant proteins, Factor IX; Tissue-type plasminogen activator; proinsulin; gamma-interferon, interleukin-2; extracellular proteins containing disulphide bonds whose action is to destroy antibiotics either enzymatically or by binding, β-lactamase inhibitor, α-trypsin inhibitor; nerve growth factors, tissue necrosis factors, colony-stimulating factors; synthetic, designed or engineered antibody molecules; cholesterol receptor; viral hemagglutinins, AIDS antigen and immunogen, hepatitis B antigen and immunogen, foot-and-mouth disease virus antigen and immunogen; protein A; protein insecticides, algicides, fungicides, and fungicides; Myocardial Infarct Protein (MIP), Weight Control Factor (WCF) or Calloric Rate Protein (CRP).

4. The method of claim 1, wherein the regulatory sequence also includes an operator sequence, a transcription-start sequence, and a ribosome-binding site sequence.

5. A method for preparing a replicable microbial expression vehicle capable of expressing an eukaryotic protein, wherein said eukaryotic protein has at least one cystine residue and has a DNA sequence according to any preceding claim.

6. A method for preparing a Streptomyces host harbouring a replicable microbial expression vehicle, which comprises introducing into the host a vehicle according to claim 5.

7. The method of claim 6, wherein the host has a disulphide-interchange enzyme or disulphide-interchange enzyme-like activity.

8. A process for producing an active eukaryotic protein that has at least one cystine residue, said process comprising cultivating the Streptomyces host of claim 6.

9. The process of claim 8, wherein said active eukaryotic protein has biological, hormonal, immunological or enzymatic activity.

## Patentansprüche (Patentansprüche für folgende(n) Vertragsstaat(en): BE, CH, DE, FR, GB, GR, IT, LU, NL, SE)

1. DNA-Sequenz, die für ein eukaryotisches Protein mit mindestens einem Cystinrest kodiert, umfassend:
(a) eine aus einem Streptomyces-Wirt ausgewählte regulatorische Nukleotidsequenz, einschließlich einer Promotor-Sequenz und einer Signalsequenz, die dafür verantwortlich ist, daß ein homologes Translationsprodukt zu dem zellulären System geschickt wird, das für die Sekretion von Disulfid-gebundenen Proteinen verantwortlich ist, funktionsfähig verknüpft mit
(b) einer zweiten Nukleotidsequenz, die für ein Disulfidbindung-enthaltendes heterologes eukaryotische Protein kodiert;
mit der Maßgabe, daß die regulatorische Sequenz nicht aus einem Streptomces-endoH-Gen ausgewählt ist.

2. DNA-Sequenz nach Anspruch 1, worin der Streptomyces-Wirt ausgewählt ist aus der Spezies acidophillus, albus, amylolyticus, argentiolus, aureofaciens, aureus, candidus, cellostaticus, cellulolyticus, coelicolor, creamorus, diastaticus, farinosus, flaveolus, flavogriseus, fradiae, fulvoviridis, fungicidicus, gelaticus, glaucescens, globisporus, griseolus, griseus, hygroscopicus, ligninolyticus, lipolyticus, lividans, moderatus, olivochromogenus, parvus, phaeochromogenes, plicatus, proteolyticus, rectus, roseolus, roseoviolaceus, scabies, thermolyticus, tumorstaticus, venezuelae, violaceus, violaceus-ruber, violascens oder viridochromogenes.

3. DNA-Sequenz nach Anspruch 1, worin das Disulfidbindungenthaltende heterologe eukaryotische Protein heterolog bezüglich des Streptomyces-Wirts ist und ein natürliches oder konstruiertes Protein ist, welches ausgewählt ist aus der Gruppe aus Prochymosin, Trypsinen, Amylasen, Ligninasen, Chymosin, Lipasen, Cellulasen; Glucoseisomerase, Pectinasen, Proteinasen, Oxidasen; Faktor VIII und Faktor VIII-verwandten biosynthetischen Blutgerinnungsproteinen, Faktor IX; Plasminogenaktivator vom Gewebetyp; Proinsulin; Gamma-Interferon, Interleukin-2; extrazellulären Disulfidbindungen-enthaltenden Proteinen, deren Wirkung darin besteht, Antibiotika entweder enzymatisch oder durch Bindung zu zerstören, β-Lactamase-Inhibitor, α-Trypsin-Inhibitor; Nervenwachstumsfaktoren, Nekrosefaktoren des Gewebes, Kolonie-stimulierenden Faktoren; synthetischen, konstruierten oder gentechnisch hergestellten Antikörper-Molekülen; Cholesterin-Rezeptor; viralen Hämaglutininen, AIDS-Antigen und -Immunogen, Hepatitis B-Antigen und -Immunogen, Antigen und Immunogen des Maul- und Klauenseuchevirus; Protein A, Protein-Insektiziden, -Algiciden, -Fungiziden, und Fungiziden; myocardialem Infarktprotein (MIP), Gewichtskontrollfaktor (WCF) oder Protein der Wärmerate (CRP).

4. DNA-Sequenz nach Anspruch 1, worin die regulatorische Sequenz auch eine Operator-Sequenz, eine Transkriptionsstart-Sequenz und eine ribosomale Bindungsstellen-Sequenz einschließt.

5. Replizierbares mikrobielles Expressionsvehikel, welches imstande ist, ein eukaryotisches Protein zu exprimieren, worin das eukaryotische Protein mindestens einen Cystinrest aufweist und eine DNA-Sequenz nach irgendeinem der vorhergehenden Ansprüche besitzt.

6. Streptomyces-Wirt, der ein replizierbares mikrobielles Expressionsvehikel nach Anspruch 5 beherbergt.

7. Streptomyces-Wirt nach Anspruch 6, der ein Disulfidaustausch-Enzym oder eine einem Disulfidaustausch-Enzym ähnliche Aktivität aufweist.

8. Verfahren zur Herstellung eines aktiven eukaryotischen Proteins, das mindestens einen Cystinrest aufweist, welches Verfahren die Züchtung des Streptomyces-Wirts nach Anspruch 6 umfaßt.

9. Verfahren nach Anspruch 8, worin das aktive eukaryotische Protein biologische, hormonelle, immunologische oder enzymatische Aktivität aufweist.

## Patentansprüche (Patentansprüche für folgende(n) Vertragsstaat(en): AT, ES)

1. Verfahren zur Herstellung einer DNA-Sequenz, die für ein eukaryotisches Protein mit mindestens einem Cystinrest kodiert, welches umfaßt die funktionsfähige Ver-knüpfung:
(a) einer aus einem Streptomyces-Wirt ausgewählten regulatorischen Nukleotidsequenz, einschließlich einer Promotor-Sequenz und einer Signalsequenz, die dafür verantwortlich ist, daß ein homologes Translationsprodukt zu dem zellulären System geschickt wird, welches für die Sekretion von Disulfid-gebundenen Proteinen verantwortlich ist, mit
(b) einer zweiten Nukleotidsequenz, die für ein Disulfidbindung-enthaltendes heterologes eukaryotisches Protein kodiert;
mit der Maßgabe, daß die regulatorische Sequenz nicht aus einem Streptomyces-endoH-Gen ausgewählt ist.

2. Verfahren nach Anspruch 1, worin der Streptomyces-Wirt ausgewählt ist aus der Spezies acidophillus, albus, amylolyticus, argentiolus, aureofaciens, aureus, candidus, cellostaticus, cellulolyticus, coelicolor, creamorus, diastaticus, farinosus, flaveolus, flavogriseus, fradiae, fulvoviridis, fungicidicus, gelaticus, glaucescens, globisporus, griseolus, griseus, hygroscopicus, ligninolyticus, lipolyticus, lividans, moderatus, olivochromogenus, parvus, phaeochromogenes, plicatus, proteolyticus, rectus, roseolus, roseoviolaceus, scabies, thermolyticus, tumorstaticus, venezuelae, violaceus, violaceus-ruber, violascens oder viridochromogenes.

3. Verfahren nach Anspruch 1, worin das Disulfidbindungenthaltende heterologe eukaryotische Protein heterolog bezüglich des Streptomyces-Wirts ist und ein natürliches oder konstruiertes Protein ist, das ausgewählt ist aus der Gruppe aus Prochymosin, Trypsinen, Amylasen, Ligninasen, Chymosin, Lipasen, Cellulasen; Glucoseisomerase, Pectinasen, Proteinasen, Oxidasen; Faktor VIII und Faktor VIII-verwandten biosynthetischen Blutgerinnungsproteinen, Faktor IX; Plasminogenaktivator vom Gewebetyp; Proinsulin; Gamma-Interferon, Interleukin-2; extrazellulären Disulfidbindungen-enthaltenden Proteinen, deren Wirkung darin besteht, Antibiotika entweder enzymatisch oder durch Bindung zu zerstören, β-Lactamase-Inhibitor, α-Trypsin-Inhibitor; Nervenwachstumsfaktoren, Nekrosefaktoren des Gewebes, Kolonie-stimulierenden Faktoren; synthetischen, konstruierten oder gentechnisch hergestellten Antikörper-Molekülen; Cholesterin-Rezeptor; viralen Hämaglutininen, AIDS-Antigen und -Immunogen, Hepatitis B-Antigen und -Immunogen, Antigen und Immunogen des Maul- und Klauenseuchevirus; Protein A, Protein-Insektiziden, -Algiciden, -Fungiziden, und Fungiziden; myocardialem Infarktprotein (MIP), Gewichtskontrollfaktor (WCF) oder Protein der Wärmerate (CRP).

4. Verfahren nach Anspruch 1, worin die regulatorische Sequenz auch eine Operator-Sequenz, eine Transkriptionsstart-Sequenz und eine ribosomale Bindungsstellen-Sequenz einschließt.

5. Verfahren zur Herstellung eines replizierbaren mikrobiellen Expressionsvehikels, das imstande ist, ein eukaryotisches Protein zu exprimieren, wobei das eukaryotische Protein mindestens einen Cystinrest aufweist und eine DNA-Sequenz nach irgendeinem der vorhergehenden Ansprüche besitzt.

6. Verfahren zur Herstellung eines Streptomyces-Wirts, der ein replizierbares mikrobielles Expressionsvehikel beherbergt, welches umfaßt, daß in den Wirt ein Vehikel nach Anspruch 5 eingeführt wird.

7. Verfahren nach Anspruch 6, worin der Wirt ein Disulfidaustausch-Enzym oder eine einem Disulfidaustausch-Enzym ähnliche Aktivität aufweist.

8. Verfahren zur Herstellung eines aktiven eukaryotischen Proteins, das mindestens einen Cystinrest aufweist, welches Verfahren die Züchtung des Streptomyces-Wirts nach Anspruch 6 umfaßt.

9. Verfahren nach Anspruch 8, worin das aktive eukaryotische Protein biologische, hormonelle, immunologische oder enzymatische Aktivität aufweist.

## Revendications (Revendications pour l'(les) Etat(s) contractant(s) suivant(s): BE, CH, DE, FR, GB, GR, IT, LU, NL, SE)

1. Séquence d'ADN codant pour une protéine eucaryote présentant au moins un résidu de cystine, et comportant: (a) une séquence de nucléotides régulante, choisie dans un hôte Streptomyces, la séquence régulante comportant une séquence de promoteur et une séquence signal responsable de l'émission d'un produit homologue de traduction vers l'appareil cellulaire responsable de la sécrétion de protéines liées par des ponts disulfure, et liées fonctionnellement à (b) une seconde séquence de nucléotides codant pour une protéine eucaryote hétérologue contenant des ponts disulfure;
avec la condition que la séquence régulante n'est pas choisie dans un gêne endoH de Streptomyces.

2. Séquence d'ADN selon la revendication 1, dans laquelle ledit hôte Streptomyces est choisi dans les espèces acidophillus, albus, amylolyticus, argentiolus, aureofaciens, aureus, candidus, cellostaticus, cellulolyticus, coelicolor, creamorus, diastaticus, farinosus, flaveolus, flavogriseus, fradiae, fulvoviridis, fungicidicus, gelaticus, glaucescens, globisporus, griseolus, griseus, hygroscopicus, ligninolyticus, lipolyticus, lividans, moderatus, olivochromogenus, parvus, phaeochromogenes, plicatus, proteolyticus, rectus, roseolus, roseoviolaceus, scabies, thermolyticus, tumorstaticus, venezuelae, violaceus, violaceus-ruber, violascens ou viridochromogenes.

3. Séquence d'ADN selon la revendication 1, dans laquelle la protéine eucaryote hétérologue contenant des ponts disulfure est hétérologue vis-à-vis dudit hôte Streptomyces et est une protéine naturelle ou de synthèse choisie dans le groupe constitué de la prochymosine, des trypsines, des amylases, des ligninases, de la chymosine, des lipases, des cellulases; de l'isomérase du glucose, des pectinases, des protéinases, des oxydases; du facteur VIII et de protéines biosynthétiques de coagulation du sang apparentées au facteur VIII, du facteur IX; d'un activateur de plasminogène du type tissulaire; de la proinsuline; du gamma-interféron, de l'interleukine-2; des protéines extracellulaires contenant des ponts disulfures et dont l'action est de détruire des antibiotiques enzymatiquement ou par liaison, d'un inhibiteur de la β-lactamase, d'un inhibiteur de l'α-trypsine; de facteurs de croissance des nerfs, de facteurs de nécrose des tissus, de facteurs de stimulation de colonies; de molécules d'anticorps obtenues par synthèse biochimique ou génétique; d'un récepteur du cholestérol; d'hémagglutinine virale, d'un immunogène et d'un antigène du SIDA; d'un immunogène et d'un antigène de l'hépatite B; d'un immunogène et d'un antigène du virus de la fièvre aphteuse; de la protéine A; d'algicides, de fongicides, de biocides et d'insecticides protéiques; de la protéine de l'infarctus du myocarde (MIP), de la protéine du facteur de contrôle de poids (WCF) ou de la protéine du taux calorique (CRP).

4. Séquence d'ADN selon la revendication 1, dans laquelle la séquence régulante comporte aussi une séquence d'opérateur, une séquence de début de transcription, et une séquence de site de liaison sur ribosomes.

5. Vecteur d'expression microbien capable de réplication, capable d'exprimer une protéine eucaryote, dans lequel ladite protéine eucaryote présente au moins un résidu de cystine et présente une séquence d'ADN selon l'une quelconque des revendications précédentes.

6. Hôte Streptomyces logeant un vecteur d'expression microbien capable de réplication selon la revendication 5.

7. Hôte Streptomyces selon la revendication 6, qui présente une enzyme d'échange de ponts disulfure ou une activité similaire à celle d'une enzyme d'échange de ponts disulfure.

8. Procédé de production d'une protéine eucaryote active qui présente au moins un résidu de cystine, ledit procédé comportant la culture de l'hôte Streptomyces la revendication 6.

9. Procédé selon la revendication 8, dans lequel ladite protéine eucaryote active présente une activité biologique, hormonale, immunologique ou enzymatique.

## Revendications (Revendications pour l'(les) Etat(s) contractant(s) suivant(s): AT, ES)

1. Procédé de préparation d'une séquence d'ADN codant pour une protéine eucaryote présentant au moins un résidu de cystine, comprenant l'étape consistant à lier fonctionnellement:
(a) une séquence de nucléotides régulante, choisie dans un hôte Streptomyces, la séquence régulante comportant une séquence de promoteur et une séquence signal responsable de l'émission d'un produit homologue de traduction vers l'appareil cellulaire responsable de la sécrétion de protéines liées par des ponts disulfure, à
(b) une seconde séquence de nucléotides codant pour une protéine eucaryote hétérologue contenant des ponts disulfure;
avec la condition que la séquence régulante n'est pas choisie dans un gêne endoH de Streptomyces.

2. Procédé selon la revendication 1, dans lequel ledit hôte Streptomyces est choisi dans les espèces acidophillus, albus, amylolyticus, argentiolus, aureofaciens, aureus, candidus, cellostaticus, cellulolyticus, coelicolor, creamorus, diastaticus, farinosus, flaveolus, flavogriseus, fradiae, fulvoviridis, fungicidicus, gelaticus, glaucescens, globisporus, griseolus, griseus, hygroscopicus, ligninolyticus, lipolyticus, lividans, moderatus, olivochromogenus, parvus, phaeochromogenes, plicatus, proteolyticus, rectus, roseolus, roseoviolaceus, scabies, thermolyticus, tumorstaticus, venezuelae*,* violaceus, violaceus-ruber, violascens ou viridochromogenes.

3. Procédé selon la revendication 1, dans lequel la protéine eucaryote hétérologue contenant des ponts disulfure est hétérologue vis-à-vis dudit hôte Streptomyces et est une protéine naturelle ou de synthèse choisie dans le groupe constitué de la prochymosine, des trypsines, des amylases, des ligninases, de la chymosine, des lipases, des cellulases; de l'isomérase du glucose, des pectinases, des protéinases, des oxydases; du facteur VIII et de protéines biosynthétiques de coagulation du sang apparentées au facteur VIII, du facteur IX; d'un activateur de plasminogène du type tissulaire; de la proinsuline; du gamma-interféron, de l'interleukine-2; des protéines extracellulaires contenant des ponts disulfures et dont l'action est de détruire des antibiotiques enzymatiquement ou par liaison, d'un inhibiteur de la β-lactamase, d'un inhibiteur de l'α-trypsine; de facteurs de croissance des nerfs, de facteurs de nécrose des tissus, de facteurs de stimulation de colonies; de molécules d'anticorps obtenues par synthèse biochimique ou génétique; d'un récepteur du cholestérol; d'hémagglutinine virale, d'un immunogène et d'un antigène du SIDA; d'un immunogène et d'un antigène de l'hépatite B; d'un immunogène et d'un antigène du virus de la fièvre aphteuse; de la protéine A; d'algicides, de fongicides, de biocides et d'insecticides protéiques; de la protéine de l'infarctus du myocarde (MIP), de la protéine du facteur de contrôle de poids (WCF) ou de la protéine du taux calorique (CRP).

4. Procédé selon la revendication 1, dans lequel la séquence régulante comporte aussi une séquence, une séquence de début de transcription, et une séquence de site de liaison sur ribosomes.

5. Procédé de préparation d'un vecteur d'expression microbien capable de réplication, capable d'exprimer une protéine eucaryote, dans lequel ladite protéine eucaryote présente au moins un résidu de cystine et présente une séquence d'ADN selon l'une quelconque des revendications précédentes.

6. Procédé de préparation d'un hôte Streptomyces logeant un vecteur d'expression microbien capable de réplication, qui comprend l'étape consistant à introduire dans l'hôte un vecteur selon la revendication 5.

7. Procédé selon la revendication 6, dans lequel l'hôte présente une enzyme d'échange de ponts disulfure ou une activité similaire à celle d'une enzyme d'échange de ponts disulfure.

8. Procédé de production d'une protéine eucaryote active qui présente au moins un résidu de cystine, ledit procédé comportant la culture de l'hôte Streptomyces de la revendication 6.

9. Procédé selon la revendication 8, dans lequel ladite protéine eucaryote active présente une activité biologique, hormonale, immunologique ou enzymatique.
